# EUROPEAN PATENT APPLICATION

(11) **EP 3 597 222 A1**
(43) Date of publication of application: **22.01.2020**
(21) Application number: 18183584.4
(22) Date of filing: 16.07.2018
(51) Int. Cl.: A61K 48/00, A61P 9/10, C12N 15/113, A61P 25/28, A61P 29/00

(54) **TREATMENT AND DIAGNOSIS OF UNRESOLVED INFLAMMATORY DISEASES**

(71) Applicant: Easemedcontrol R & D GmbH & Co KG, 80779 München (DE)
(72) Inventor: The designation of the inventor has not yet been filed
(74) Representative: Dr. Langfinger & Partner

(57) **Abstract**

A compound for use in the treatment or prevention or diagnosis of a disease or condition in a mammalian subject selected from unresolved inflammatory conditions wherein the compound is a modulator of the expression, function or stability of C1q or of the interaction of ApoE with C1q or of ApoE with Aβ or of complement component C5 or its cleavage products C5a and C5b or variants thereof or of any complement constituent that arises between the C1q complex and C5 or cleavage products thereof or complement receptors on any cell and wherein said disease is selected from unresolved inflammatory conditions, a method for identifying such compound, diagnostic methods for the diagnosis of aging-associated diseases and isolated antigen binding constructs .

## Description

Apolipoprotein E (hereinafter ApoE) is a polymorphic protein arising from three alleles at a single gene locus. It is a multidomain lipid-binding protein largely produced by the liver but neuronal cells and - in particular - brain cells including microglia cells can produce ApoE under stress conditions as well (Mahley, 1988; Mahley et al., 2009).

The human isoforms of ApoE, i.e. ApoE2, ApoE3 and ApoE4 differ in amino acid residues 112 and 158 located outside the N-terminal receptor- and C-terminal lipid-binding domains, respectively, thereby yielding proteins with different impacts on tissue homeostasis (Bell et al., 2012; Holtzman et al., 2012; Kanekiyo et al., 2014; Linton et al., 1995; Mahley and Huang, 2012; Plump et al., 1992; Zlokovic, 2013).

ApoE, depending on its isoform, its cellular source, the nature of the lipid moiety it binds and further multiple genetic and environmental risk factors, can act through multiple pathways. Notions regarding mechanisms of action of ApoE include isoform-specific domain-domain interactions (Zlokovic, 2013); involvement of lipoprotein receptors (Holtzman et al., 2012; Kanekiyo et al., 2014); effects on cholesterol efflux (Mahley and Huang, 2012); maintenance of the blood brain barrier (BBB) (Bell et al., 2012; Zlokovic, 2013); and binding extracellular molecules including beta amyloid peptide and heparan sulfate proteoglycans (Holtzman et al., 2012). This wide range of activities indicates that ApoE exercises its functions in complex territorialized tissue contexts.

ApoE has been implicated in a number of disease conditions, including Alzheimer's disease (hereinafter AD), other degenerative and inflammatory brain diseases, cancer, and atherosclerosis. AD is the most common form of dementia and the ApoE4 isoform predisposes to late onset AD (LOAD) and sporadic AD whereas the ApoE2 isoform protects from LOAD and sporadic AD. Moreover, the presence of one or two copies of the E4 allele has been found to increase the risk for LOAD or for sporadic AD in a gene dose-dependent way whereas the E2 allele has been associated with a decreased risk for LOAD or sporadic AD (Holtzman et al., 2012; Kanekiyo et al., 2014); these data indicate that ApoE concentrations in tissues are critical to maintain tissue homeostasis to achieve a balance between a physiological level and a disease-associated level. Atherosclerosis is the leading cause of death worldwide and AD is the leading cause of dementia and cognitive decline worldwide. In addition, patients with atherosclerosis and AD show a marked synergistic incidence of dementia indicating shared mechanisms between atherosclerosis and AD pathogeneses (Hofman et al., 1997).

Multiple clinically significant diseases as diverse as cancer, transplant rejection, atherosclerosis, age-related macular degeneration (AMD, i.e. the major cause of blindness in the elderly), renal diseases and multiple degenerative as well as autoimmune diseases have been associated with activation of one or more complement pathways, i.e. and one or more than one of a) the classical complement cascade (CCC); b) the alternative pathway; and c) the lectin pathway (Brennan et al., 2016; Hajishengallis et al., 2017).

The complement system is an important part of the immune system that enhances (complements) the ability of antibodies and phagocytic cells or other immune cells to clear microbes and damaged cells from an organism, promotes inflammation, and attacks pathogens in multiple ways including inflicting their death via the terminal complement complex (TCC). The complement system also has multiple additional effects on the immune system and activates inflammasomes whose action is presently the focus of international research efforts (Hajishengallis et al., 2017; Hess and Kemper, 2016). The complement system consists of a number of small proteins found in the blood, many of which are synthesized by the liver. Complement constituents normally circulate as inactive precursors (precursor complement proteins, also referred to frequently as pro-proteins). The complement system becomes tissue-bound under distinct disease or acute repair conditions. When stimulated by one of several triggers, proteases in the system cleave specific proteins to release downstream molecules and initiate an amplifying cascade of further cleavages. The result of this activated complement cascade is stimulation of immune cells to clear foreign and damaged material, proxy inflammation to attract additional immune cells, and formation and activation of the cell-killing TCC. New functions of the complement systems are emerging through recent research: These functions are largely beneficial but can turn into disease-relevant and disease-promoting impacts in unresolvable inflammatory diseases requiring therapy (Brennan et al., 2016). For example, the complement system plays important physiological roles as evidenced by synapse pruning during normal development of the brain, it may act intracellularly, and it skews the immune system into distinct directions either in beneficial or detrimental ways (Kolev et al., 2014; Stevens et al., 2007). In unresolvable inflammation, however, overactivation of the CCC and other complement pathways causes disease.

CCC activation is triggered by activation of the C1 complex. The C1-complex is composed of one hexameric C1q molecule (comprising 2 x C1qa, 2 x C1qb, and 2 x C1qc proteins), two molecules of C1r and two molecules of C1s, referred to as *C1qr²s².* This occurs when C1q binds to immunoglobulins such as IgM or IgG complexed with antigens, surfaces or by other means by a variety of activators. This also occurs when C1q binds directly to the surface of the pathogen or other activating surfaces. Such binding leads to conformational changes in the C1q molecule, which leads to the activation of the C1r protease, which then cleaves C1s protease. The C1r²s² complex now splits C4 and then C4b-bound C2, producing C4a, C4b, C2a, and C2b. C4b and C2b (historically, the larger fragment of C2 was called C2a but is now referred to as C2b) assemble to form the C3-convertase (C4b2b complex), which promotes cleavage of C3 into C3a and C3b. C3b later joins with C4b2b to generate C5 convertase (C4b2b3b complex). Several functions have been assigned to C1q, including CCC-dependent and CCC-independent immune functions (Hajishengallis et al., 2017; Kolev et al., 2014; Kouser et al., 2015).

Complement component 5 (hereinafter C5) is the fifth component of complement, which plays an important role in inflammation and cell killing. This protein is composed of alpha and beta polypeptide chains that are linked by a disulfide bridge. An activation peptide, C5a, which is an anaphylatoxin that possesses potent spasmogenic and chemotactic activity, is derived from the alpha polypeptide via cleavage by a C5-convertase. The C5b macromolecular cleavage product can form a complex with the C6 complement component, and this complex is the basis for formation of the TCC which is also referred to as the membrane attack complex (MAC), which includes additional complement components (Brennan et al., 2016; Hajishengallis et al., 2017).

Eculizumab, sold under the trade name Soliris, is a medication used to treat the diseases, i.e. paroxysmal nocturnal hemoglobinuria (PNH) and atypical hemolytic uremic syndrome (aHUS). It is a humanized monoclonal antibody functioning as a terminal complement inhibitor, having complement protein C5 as target. Eculizumab inhibits terminal complement activation and therefore makes people vulnerable to infection with encapsulated organisms, in particular to meningococcal infections (Brennan et al., 2016).

Small interfering RNA (siRNA) is a class of double-stranded RNA molecules having 20-25 base pairs in length which operates in the RNA interference pathway. It interferes with the expression of specific genes with complementary nucleotide sequences by degrading mRNA after transcription, thereby preventing translation. The availability to knock-down in essence any gene of interest via siRNAs have triggered significant interest. SiRNAs have been shown to have therapeutic potential for central nervous system disorders. US 2017/183659 discloses antisense nucleotide agents (e.g. si-RNAs) for inhibiting expression of complement component C5 wherein the agent comprises 4 to 50 nucleotides, wherein all nucleotides or a part thereof may be modified nucleotides. The agents are used for treating disorders which benefit from inhibiting or reducing the expression of a C5 mRNA such as PNH and aHUS.

EP 3034510 discloses oligonucleotides which may be siRNAs for the treatment of unwanted immune responses, inflammatory diseases and neurological diseases.

WO2016/044419 and WO2016/201301 disclose double stranded ribonucleic acid agents for inhibiting expression of complement component C5 and pharmaceutical compositions comprising said agents for treatment of a variety of diseases which benefit from reduction or inhibition of the expression of complement component C5, including PNH and aHUS, atherosclerosis and AD.

Amyloid β (Aβ) forms insoluble aggregates in the brain and there it is a pathological hallmark of AD plaques.

WO2011/109246 discloses a method of reducing binding of apolipoprotein E4 (apoE4) to an amyloid-β -peptide (e.g. an amyloid plaque) in the brain of an individual (thereby treating AD) which comprises administering a synthetic antibody specifically binding to an epitope in apoE.

US 2015/0337030 relates to methods of treating, preventing or delaying the onset of sporadic AD in a subject, the methods comprising administering to the subject a therapeutically effective amount of an antibody that binds to the ApoE4 protein or a peptide or a peptidomimetic that binds to the ApoE4 protein, or an antisense RNA or an si-RNA that inhibits expression of the gene that encodes the ApoE4 protein.

Brennan et al. summarize work done on complement inhibition in AD (Brennan et al., 2016). It is outlined that C1q is abundantly present in senile plaques and that a predominantly detrimental role for C1q has been initially proposed. Later findings, however, suggested that complement proteins can also have positive roles in AD (Hajishengallis et al., 2017).

### Brief description of the Figures

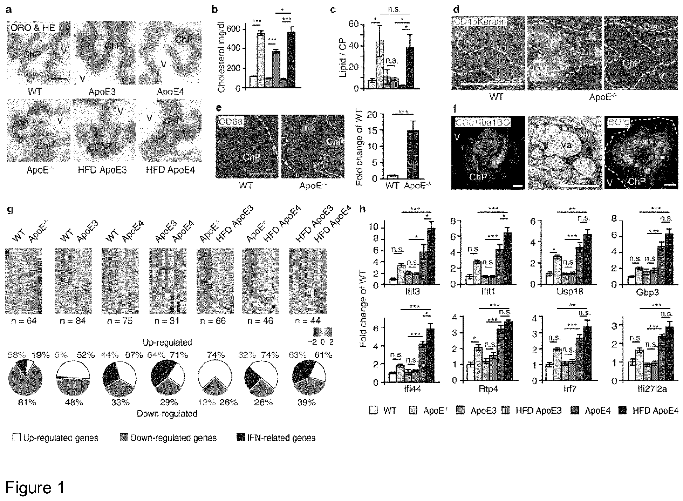

Figure 1 shows ChP lipid, inflammation and interferon (IFN) signatures. (a) For ChP-Lipid determination ChP sections were stained with ORO/HE. Bar 100 µm; (b) plasma cholesterol. WT (n=6); ApoE^{-/-}(n=6); ApoE3 (n=14); ApoE3 HFD (n=17); ApoE4 (n=8); ApoE4 HFD (n=10) mice; (c) Quantification of lipid⁺ areas. Lipid⁺ areas in ChPs and associated parenchyma per tissue area were quantified; WT (n=3); ApoE^{-/-} (n=9); ND ApoE3 (n=6); HFD ApoE3 (n=6); ND ApoE4 (n=6); HFD ApoE4 (n=9); Means ± SEM, **P*<0.05, ***P*<0.01, ****P*<0.001; one-way ANOVA with Tukey posttest for b and c; (d) leukocytes in ChP; epithelial cells were stained for cytokeratin (Keratin), leukocytes (CD45), and nuclei by DAPI (4'6-diamidino-2-phenylindole) . Phase contrast delineates the ChP. Dashed line indicates the border of ChP and the ventricle. Bar 100 µm; (e) macrophage/DC (CD68). CD68⁺ areas were quantified. Two-tailed Student's t-test, ****P*<0.001. 12 sections from 4 WT; 12 sections from 4 ApoE^{-/-} mice; (f) ChPs were stained for lipid by BODIPY (4,4-difluoro-1,3,5,7,8-pentamethyl-4-bora-3a,4a-diaza-s-indacene, BO), endothelial cells (CD31), and macrophages (lba-1) (left panel); TEM shows a single ChP macrophage-foam cell (middle panel); and lipid (BO) and immunoglobulin (Ig) (right panel). Bars 10 µm; (g) IFN-related genes in ChPs by microarray. Heatmaps show two-group comparisons of ChPs. The percentages of up- or down-regulated genes are shown in the circle. (h) ApoE4 isoform-dependent IFN signature expression in ChPs. Abbreviations used: ChP, choroid plexus; V, ventricle; Ep, epithelial cells; Nu, nucleus; Va, vacuole; TJ, tight junction.

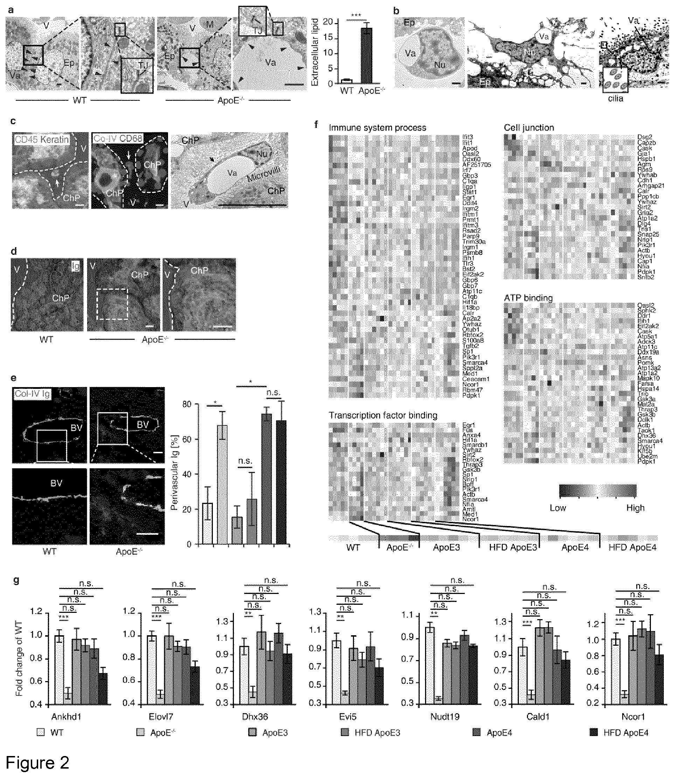

Figure 2 shows lipid deposits, BBB, and ChP gene signatures. (a) Lipid deposits in ChPs. Vacuole (Va) represents lipid. TEM shows intercellular lipid between two epithelial cells and intracellular lipid in epithelial cells of ApoE^{-/-} and WT mice (arrows indicate cell surface membranes of epithelial cells) Two-tailed Student's t-test, ****P*<0.001. 68 intercellular spaces from 3 ApoE^{-/-} and 67 intercellular spaces from 3 WT mice were analyzed. Bar represents 1 µm; (b) Lipid in ApoE^{-/-} ChPs by TEM. Lymphocytes (left panel); macrophages/dendritic cells (DC) (middle panel); and ependymal cells containing lipid (right panel). Vacuole (Va) represents lipid. Bar 1 µm; (c) Leukocytes at the abluminal side of ApoE^{-/-} ChPs. ChPs were stained for cytokeratin (Keratin,) and leukocytes (CD45) (left panel); collagen IV (Co-IV) and CD68 (middle panel). TEM shows single macrophage-foam cell/DC surrounded by microvilli which mark the abluminal side of epithelial cells in aged ApoE^{-/-} ChP/ventricle. Bar 10 µm; (d) Ig in ChPs of ApoE^{-/-} mice. Brains were stained with Ig. Bars 10 µm; (e) BBB breakdown in aged ApoE^{-/-} but not WT mice. PFA-perfused brains were stained for Ig (Ig) and blood vessels (Col-IV) in the cerebellum. Perivascular Ig adjacent to blood vessels was quantified. WT (3); ApoE^{-/-}(3); ND ApoE3 (3); HFD ApoE3 (3); ND ApoE4 (3); HFD ApoE4 (3). Bar 10 µm. Results represent means ± SEM; two-tailed Student's t-test **P*<0.05, ****P*<0.001, n.s. = not significant; (f) Laser capture microdissection (LCM)-based expression microarrays of ChPs. Heatmaps show transcript levels in GO terms *immune system process, transcription factor binding, cell junction,* and *ATP binding*; (g) Rescued gene expression levels in ApoE-expressing mice. Genes that were down-regulated in ApoE^{-/-} ChPs and rescued either in ApoE3-KI and in ND or HFD ApoE4-KI mice. Means ± SEM, **P*<0.05, ***P*<0.01, ****P*<0.001, one-way ANOVA with Tukey posttest, WT (5); ApoE^{-/-} (4); ND ApoE3 (6); HFD ApoE3 (6); ND ApoE4 (6); HFD ApoE4 (6).

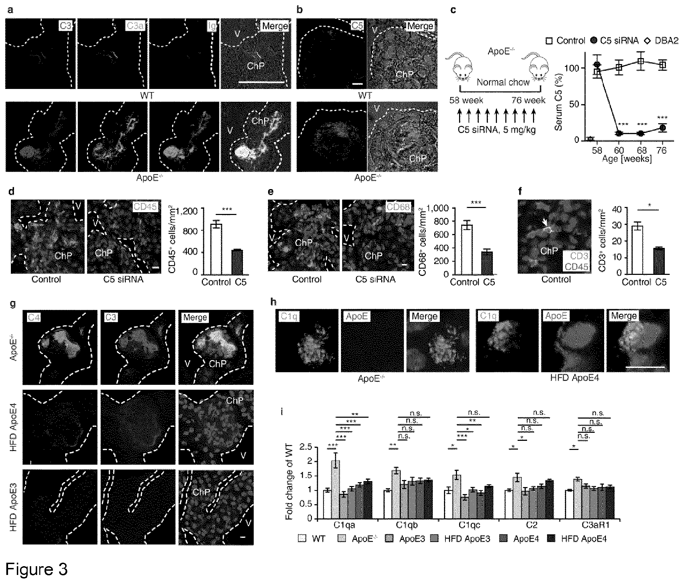

Figure 3 shows how complement affects ChP inflammation. C3 and C3a in ChPs. ChPs were stained for complement C3, anaphylatoxin C3a, and Ig. Bar 100 µm. (b) Complement C5. ChPs were stained for complement C5. Bar 10 µm. (c) Liver-targeted C5-siRNA reduces serum C5. (d-f) C5 siRNA attenuates leukocyte infiltration (d), CD68⁺ macrophage/DC infiltration (e), and CD3⁺ T cell infiltration (f) in ApoE^{-/-} ChPs. Bars 10 µm. Data represent means ± SEM, two-tailed Student's t-test. **P*<0.05, ****P*<0.001, Control (6), C5 (6). (g) Low C4 and C3 protein levels in lipid deposits of HFD ApoE4 ChPs. Serial sections of ChPs as shown in Fig.1a were stained for C4 and C3. (h) C1q and ApoE colocalize in HFD ApoE4-KI ChPs. Super-resolution (STED) microscopy shows colocalization of C1q and ApoE in HFD ApoE4-KI ChPs. Bar 10 µm. (i) ChP complement mRNA expression. Means ± SEM, **P*<0.05, ***P*<0.01, ****P*<0.001; one-way ANOVA with Tukey posttest, WT (n=4); ApoE^{-/-}(n=5); ND ApoE3 (n=6); HFD ApoE3 (n=6); ND ApoE4 (n=6); HFD ApoE4 (n=6).

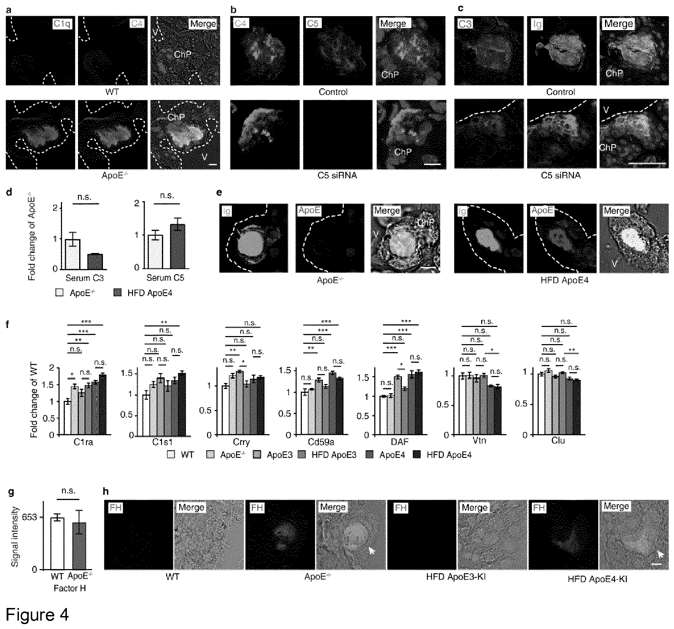

Figure 4 shows complement constituents in mouse ChPs. (a) CCC constituents. ChPs were stained for C1q and C4. Bar 100 µm. (b) C5 siRNA treatment blocks C5 protein deposition in ApoE^{-/-} ChPs; (c) ChPs were stained for C3. Ig is a proxy for lipid; (d) Serum C3 and C5. Serum C3 and C5 protein levels were measured by ELISA. Means ± SEM, n.s., not significant. Two-tailed Student's t-test. ApoE^{-/-}(n=6), HFD ApoE4 (n=5). (e) High resolution confocal microscopy shows colocalization of ApoE4 (ApoE) and Ig (represents lipid) in HFD ApoE4-KI ChPs. ApoE^{-/-} ChPs serve as negative controls for ApoE staining; (f) Complement regulators are expressed in ChPs. Means ± SEM, **P*<0.05, ***P*<0.01, ****P*<0.001, one-way ANOVA with Tukey posttest, WT (n=5); ApoE^{-/-}(n=4); ND ApoE3 (n=6); HFD ApoE3 (n=6); ND ApoE4 (n=6); HFD ApoE4 (n=6). (g) Factor H is expressed WT and ApoE^{-/-} ChPs. n.s. = not significant. Two-tailed student t test, WT (n=5); ApoE^{-/-}(n=4); (h) ChP factor H protein in ChPs. White arrows indicate lipid positive areas.

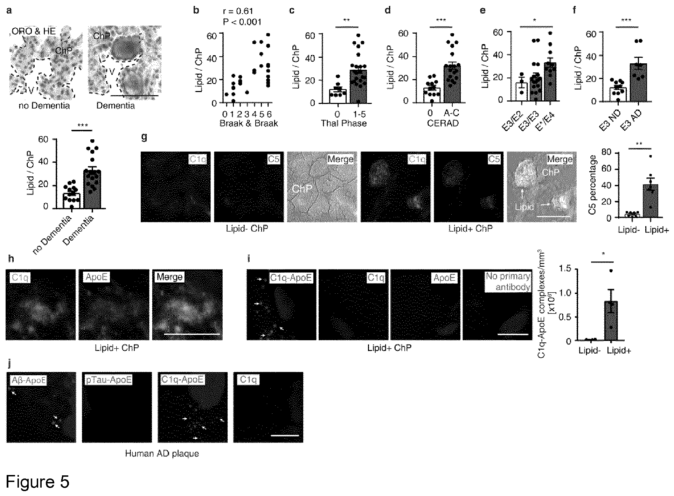

Figure 5 shows correlation of ChP C1q-ApoE complexes with cognitive decline in Alzheimer's disease (AD). (a) Human ChP sections were stained with ORO/HE. Bar 100 µm. ChPs lipid was quantified. Non-dementia cases (n=13) and demented cases (n=17). ****P*<0.001. Two-tailed Student's t-test. (b) Pearson correlation of ChP lipid and neurofibrillary tangle stage (Braak & Braak). n=30, r=0.61, *P*<0.001. (c-e) ChP lipid correlate with Aβ score (Thai phase) (c), neuritic plaque score (CERAD) (d), and ApoE4 genotype (e). **P*<0.05, ***P*<0.01, ****P*<0.001. Two-tailed Student's t-test (c,d) and One-way ANOVA (e). (f) ChP lipid correlates with dementia in ApoE3/ApoE3 carriers. ***P*<0.01, Two-tailed Student's t-test. ApoE3/ApoE3 Non-dementia cases (n=10) and demented cases (n=7). (g) Human ChP sections were stained for C1q and C5. Bar 100 µm. C5 percentage of lipid- ChP and lipid+ ChP from the same case was quantified. ***P*<0.01, Paired-two-tailed Student's t-test. n=7. (h) STED microscopy shows colocalization of C1q and ApoE. Bar 5 µm. (i) Binding of C1q-ApoE in vivo by PLA. Anti-ApoE, anti-C1q, or no primary antibodies were used as controls. The number of C1q-ApoE complexes of lipid-negative ChP or lipid-positive areas were quantified as described in Methods. **P*<0.05, Paired two-tailed Student's t-test. n=4. Bar 5 µm. (j) Human brain sections were stained for Aβ/ApοE, pTau/ApoE, C1q/ApoE, or C1q alone. Protein-protein binding in vivo was detected by PLA. Bar 5 µm.

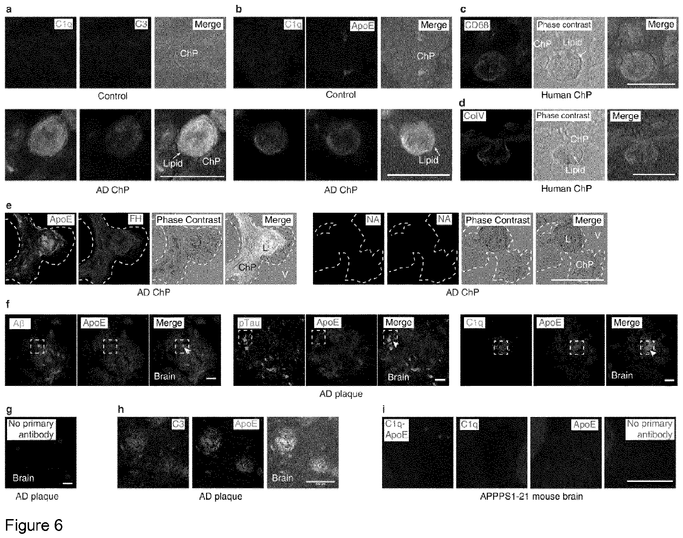

Figure 6 shows complement constituents in human ChPs. (a-b) Human ChP sections were stained for C1q / C3 (a) and C1q / ApoE (b); (c-d) ChP sections were stained for CD68⁺ macrophages/DCs (c) and collagen IV (Col-IV) to mark basement membrane. Phase contrast show lipid deposits in ChPs; (e) ChP sections were stained for ApoE and factor H, no primary antibody as control (NA). Bar 100 µm for a-e; (f-g) human brain sections were stained for Aβ / ApoE (left panels), Tau phosphorylation (pTau) / ApoE (middle panels), and C1q / ApoE (right panels) (f). No primary antibody as control (g); (h) brain parenchyma sections were stained for C3 / ApoE. Bar 100 µm; (i) C1q-ApoE complexes in mouse AD brain. 14-month old APPPS1-21 mouse brain sections were stained with C1q and ApoE. C1q-ApoE complexes were determined by PLA. ApoE alone, C1q alone, or no primary antibodies were negative controls. Bar 10 µm.

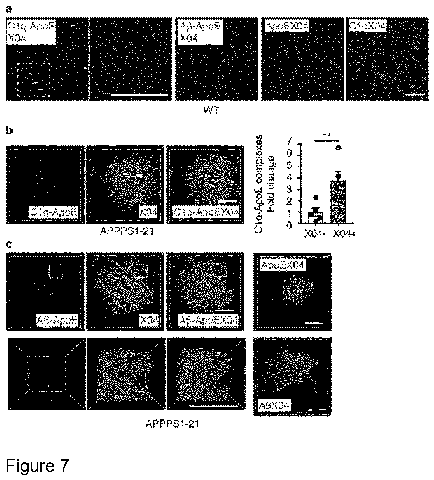

Fig 7 shows that C1q-ApoE and Aβ-ApoE complexes are hallmarks of WT and APPPS1-21 mouse brain and AD plaques, respectively. (a) 8 weeks C57BL6 (APPPS1-21) brain cortex sections were examined for the presence of C1q-ApoE complexes (white arrows) with methoxy X04 (X04, blue; fluorescent dye to label Aβ plaque). Aβ/ApoE complexes were not detected by the proximity ligation assay (PLA) using both antisera against Aβ and ApoE; antisera against Aβ or ApoE only were used as negative controls. Bars represent 10 µm. (b-c) 16 weeks AD (APPPS1-21+/-) brain cortex sections were examined by the PLA assay for the presence of C1q/ApoE complexes (b), Aβ/ApoE (c) complexes were stained with methoxy X04 to outline plaques. C1q-ApoE complexes were observed inside and in the immediate vicinity of Aβ plaque (X04+;X04-), respectively; Data represent means ± SEM, paired Student-T test, * P <0.05, n=5 mice. ApoE or Aβ only antisera were used as negative controls (c) (right two panels). High resolution confocal images show the spatial location of Aβ-ApoE complexes and Aβ plaque in 3D view (c, lower panel). Bars represent 10 µm.

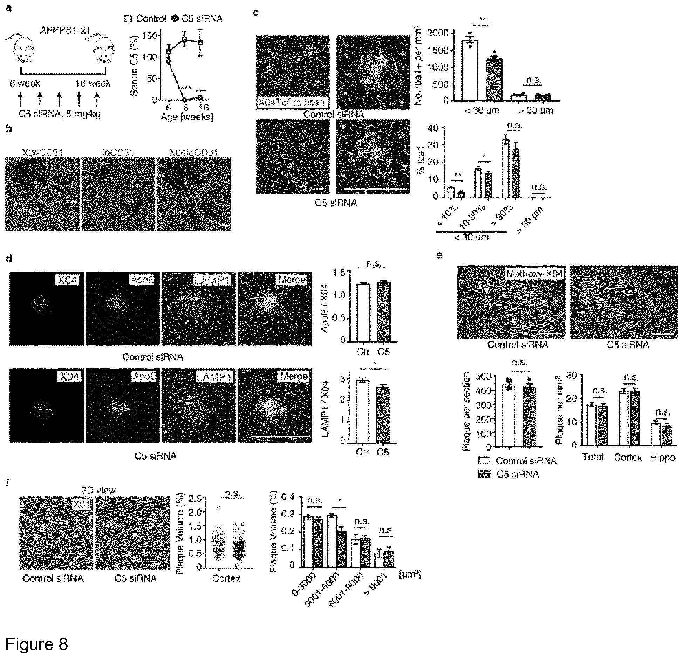

Fig. 8 shows that treatment using liver-specific C5 siRNA reduces Aβ - associated microglia cells in the brain of APPPS1-21 mice. (a) Experimental design. Serum C5 was determined by ELISA in AD mice. Data represent means ± SEM, two-tailed Student's t-test. ****P*<0.001, Control (4), C5 (5). (b) Brain sections of APPPS1-21 mice were stained with methoxy X04 for Aβ plaque, Ig for circulating immunoglobulins, and CD31 for endothelial cells. Bars 10 µm; (c) Brain sections were stained with iba1 for microglia cells (red), To-Pro-3 for nuclei, and X04 for Aβ plaques. The number of iba1+/To-Pro-3+ cells per plaque (<30 µm radius) or per area (>30 µm radius) represent data outside/in the vicinity of the Aβ plaque area. Plaques were further grouped into small plaques (X04% <10% of 30 µm radius area), moderate plaques (X04% between 10-30% 30 µm radius area), and large plaques (X04% >30% of 30 µm radius area). Percentage of X04 and iba1 of Aβ plaque areas and non-Aβ plaque areas were compared. Data represent means ± SEM, Two-way ANOVA, **P*<0.05, ***P*<0.01, n.s. = not significant. 420 individual Aβ plaques and 40 fields of non-Aβ plaques from 4 control mice, 536 individual Aβ plaques and 51 fields of non-Aβ plaques from 5 C5 siRNA-treated mice. (d) Brain sections were stained with methoxy-X04, ApoE, and LAMP1, the size of methoxy-X04, ApoE, LAMP1 were measured. ApoE/X04 and LAMP1/X04 (X04 > 150 µm²) were quantified. Two tailed Student t test. n.s. = not significant. *P < 0.05. N = 123 plaques from 4 control mice, 147 plaques from 5 C5 treated mice. Bars 100 µm. (e) Aβ plaques were stained with methoxy X04 (X04). Number of plaques per section and number of plaques per area were quantified. Data represent means ± SEM, two-tailed Student's t-test. *P*> 0.05, control (4), C5 (5). Bar 1000 µm; (f) 3D reconstruction of Aβ plaque from cortex of APPPS1-21 mice. Total plaque volumes were quantified; plaques were further grouped according to the plaque volume. Data represent means ± SEM, two-tailed Student's t-test. *P*<0.05, Control (71), C5 (88). Bar 100 µm.

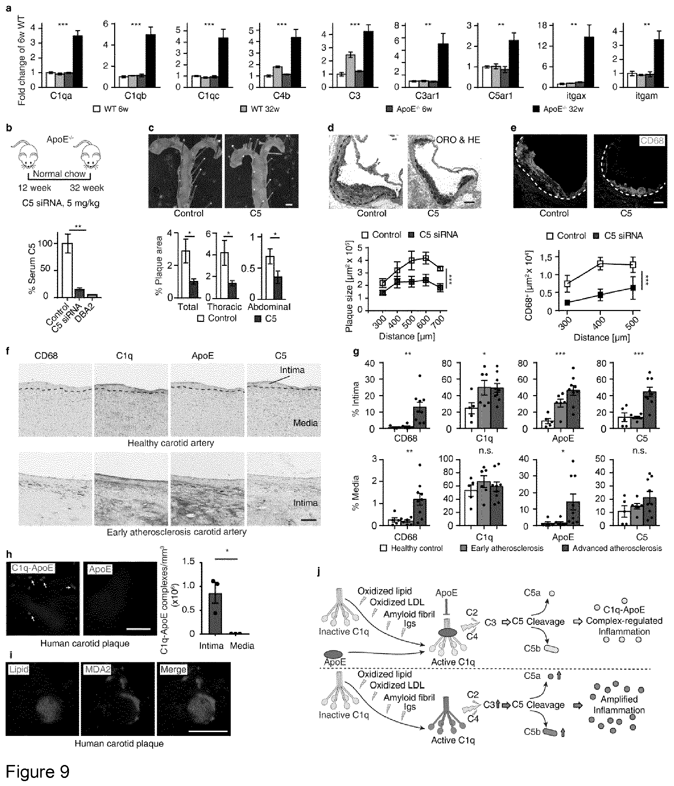

Figure 9 shows that C1q-ApoE complexes are pathological hallmarks of atherosclerosis. (a) Aorta complement gene mRNA expression. Means ± SEM, ***P*<0.01, ****P*<0.001; one-way ANOVA. 6 weeks WT (n=3); 32 weeks WT (n=3); 6 weeks ApoE^{-/-} (n=3); 32 weeks ApoE^{-/-} (n=3); (b) Liver-targeted C5 siRNA reduces serum C5 in young ApoE^{-/-} mice. (c) *En face* staining for whole aorta. Bar 0.5 cm. Control (n=11), C5 siNRA (n=12). **P*<0.05, two-tailed Student's t-test. (d-e) Aortic root sections were stained for ORO/HE and CD68⁺ macrophages/DCs. Bars 100 µm. Plaque size (d) and CD68⁺ macrophages/DCs size (e) were quantified. Control (n=4), C5-siRNA (n=4). ****P*<0.001, Two-way ANOVA. (f) Human carotid artery parallel sections were stained for CD68, C1q, ApoE, and C5 by DAB and hematoxylin. (g) CD68, C1q, ApoE, and C5 signal was quantified. **P*<0.05, ***P*<0.01, ****P*<0.001; one-way ANOVA. Control (n=5), early plaque (n=6), advanced plaque (n=9). (h) C1q-ApoE complexes in human atherosclerosis plaques were determined by PLA. **P*<0.05, Paired two-tailed Student's t-test. n=3. Bar 5 µm. (i) High resolution microscopy shows colocalization of lipid (green) and malondialdehyde epitopes (MDA2) in human atherosclerotic plaque. Bar 10 µm. (j) Schematic representation of the C1q-ApoE complex. Locally produced and/or serum-recruited C1q is activated *in situ* by a variety of surface activators including oxidized lipid, oxidized LDL, amyloid fibrils, and immunoglobulins. C1q activators have been implicated in diseases as varied as atherosclerosis and AD. Following activation, C1q acquires a conformation that allows binding of ApoE to C1q and forms the C1q-ApoE complex (upper part of the panel) which allows initiation of the CCC with resultant C3 generation, i.e. generation of C3a and C3b and C5 cleavage to generate C5a and C5b in a complex-regulated way. By contrast, inflammation is amplified in the absence of ApoE by overactivation of the CCC (lower panel).

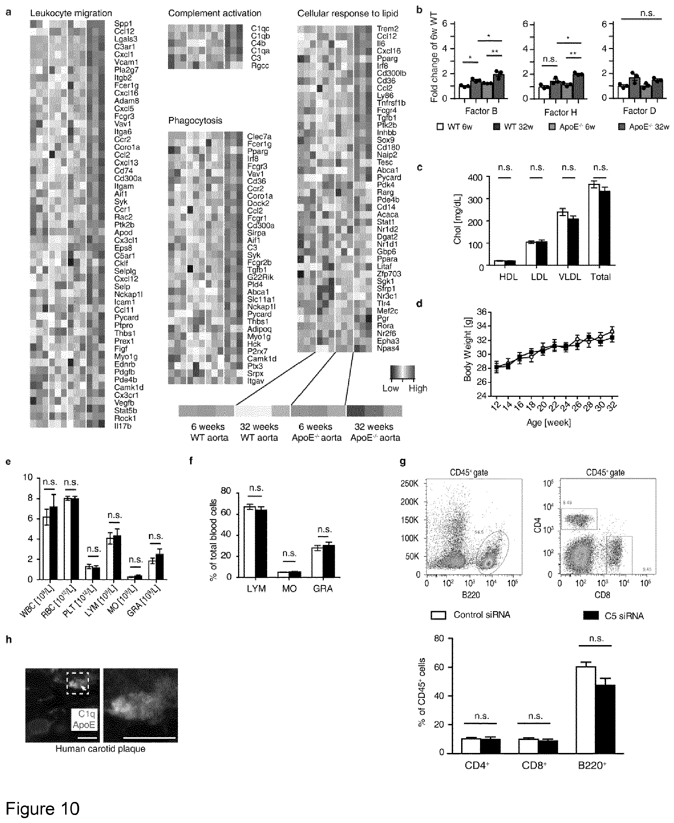

Figure 10 shows correlation of complement constituents and atherosclerosis. (a) Expression microarray analyses of aorta. Heatmaps show GO terms *leukocyte migration, complement activation, phagocytosis, and cellular response to lipid.* 6 weeks WT (n=3); 32 weeks WT (n=3); 6 weeks ApoE^{-/-} (n=3); 32 weeks ApoE^{-/-} (n=3); (b) aorta alternative complement pathway genes (factor B, factor H, factor D) mRNA expression in 6 weeks and 32 weeks old WT and ApoE^{-/-} mouse aorta. Means ± SEM, **P<0.01, ***P<0.001; one-way ANOVA with Tukey posttest. 6 weeks WT (n=3); 32 weeks WT (n=3); 6 weeks ApoE^{-/-} (n=3); 32 weeks ApoE^{-/-} (n=3); (c-d) plasma cholesterol and body weight; (e-f) blood leukocyte concentration and percentage. For c-f, control (n=11); C5 siRNA (n=12). Two-tailed Student's t-test. n.s.= not significant; (g) blood CD4⁺ T cells, CD8⁺ T cells, and B220⁺ B cells by flow cytometry. Control (n=6); C5-siRNA (n=6). Two-tailed Student's t-test. n.s.= not significant; (h) super-resolution microscopy shows colocalization of C1q and ApoE in human atherosclerotic plaque. Bar 5 µm. Abbreviation, WBC, white blood cells; RBC, red blood cells; PLT, platelets; LYM, lymphocytes; MO, monocytes; GRA, granulocytes.

There exists a need for the diagnosis and therapy of diseases involving unresolved inflammation such as AD, atherosclerosis or aging-associated diseases in general in addition to a large number of other unresolvable inflammatory diseases including cancer and diseases that can otherwise be termed unresolvable. It is therefore an object of the present invention to provide such new diagnostics and targets and to employ these methods and targets in the development of new and effective therapies. Further objects of the present invention will become apparent to the skilled person upon reading the following detailed description of the present invention. Diagnostic methods including imaging technologies of AD, atherosclerosis, and other inflammatory conditions are needed to provide in vivo procedures to develop preventive therapies.

This object is achieved in a first embodiment of the present invention with compounds for use in accordance with claim 1. Preferred embodiments are set forth in the dependent claims.

A further embodiment of the present invention is a method for identifying a compound suitable for the prevention or treatment of a disease characterized by an unresolved inflammation condition in accordance with claim 7.

A still further embodiment of the present invention is a diagnostic method for the diagnosis of aging-associated diseases, in particular AD and atherosclerosis or other forms of dementia or inflammatory brain disease, wherein the level of a complex resulting from the interaction of C1q with ApoE in the choroid plexus is monitored by an imaging method.

Isolated antigen binding construct capable of specifically binding to an interaction of ApoE and C1q or of ApoE with Aβ or capable of modulating the expression of complement component C5 or its components C5a and C5b or variants thereof of any complement constituent that arises between the C1q complex and C5 or its cleavage product or complement receptors are another embodiment of the present invention.

Pharmaceutical compositions comprising a compound for use in accordance with any of claims 1 to 6 or an isolated antigen binding construct in accordance with claim 11 and a pharmaceutically acceptable carrier, stabilizer or excipient are a further subject of the present invention.

The present invention provides a compound for use in the treatment or prevention of a disease or condition in a mammalian subject wherein the compound is a modulator (antagonist or agonist) of the expression, function or stability of the interaction of ApoE with C1q or of ApoE and Aβ or of complement component C5 or its cleavage products C5a and C5b or variants thereof or of any complement constituent that arises between C1q and the C5 complement constituent and its cleavage products or complement receptors on immune cells or tissue cells and wherein said disease is selected from unresolved inflammatory conditions.

An inflammatory response is involved from the early stages of disease development. Inflammation is a normal component of host defence, but unresolved or chronic inflammation is a core perturbation in a range of chronic diseases. In cancer, inflammation accompanies tumor growth and may be beneficial or detrimental. Upon a tissue injury (e.g. an infection for example), a variety of biological mediators trigger an acute inflammatory response in and by immune cells or tissue cells in which inflammatory molecules are involved. When the source of injury is eliminated by this process, immune agents withdraw leading to restoration of tissue homeostasis. If the source of the injury persists, chronic inflammatory diseases, i.e. conditions designated herein as unresolved inflammatory conditions, follow (Mohanta et al., 2014).

In accordance with a preferred embodiment of the present invention, the compound is an inhibitor or antagonist or agonist of said expression, function or stability of C1q or of the interaction of ApoE with C1q or of ApoE with Aβ or of complement component C5 or its cleavage products C5a and C5b or variants thereof or of any complement constituent that arises between the C1q complex and C5 or cleavage products thereof or complement receptors expressed on any cell (Brennan et al., 2016).

In accordance with another embodiment the compound is an antigen-binding construct e.g. an antibody or an antibody-like molecule or other antigen-binding derivative or an antigen-binding fragment thereof that binds said C1q or said interaction of ApoE with C1q or of ApoE with Aβ or complement component C5 or its cleavage products C5a and C5b or variants thereof or any complement constituent that arises between the C1q complex and C5 or cleavage products thereof or complement receptors on any cell .

The term "antigen binding construct" includes all varieties of antibodies, including binding fragments thereof. Further included are constructs that include 1, 2, 3, 4, 5, and/or 6 complementarity-determining regions (CDRs). In some embodiments, these CDRs can be distributed between their appropriate framework regions in a traditional antibody. In some embodiments, the CDRs can be contained within a heavy and/or light chain variable region. In some embodiments, the CDRs can be within a heavy chain and/or a light chain. In some embodiments, the CDRs can be within a DNA sequence encoding a single peptide chain. In some embodiments, the CDRs can be within two or more peptides that are covalently linked together. In some embodiments, they can be covalently linked together by a disulfide bond. In some embodiments, they can be linked via a linking molecule or moiety. In some embodiments, the antigen binding proteins are non-covalent, such as a diabody and a monovalent scFv. Unless otherwise denoted herein, the antigen binding constructs described herein bind to the noted target molecule.

The compound for use in accordance with the present invention may in one embodiment of the present invention be a nucleic acid, in particular a si-RNA (small interference RNA) or an anti-sense nucleotide molecule and/or shRNA molecule that binds to a nucleic acid that encodes or regulates the expression of (i) C1q or the interaction of ApoE with C1q or of ApoE with Aβ or complement component C5 or of complement component C5 or its cleavage products C5a and C5b or variants thereof or any complement constituent that arises between the C1q complex and C5 or cleavage products thereof or one or more complement constituent receptors on any cell or (ii) a gene that controls the expression, function and/or stability of C1q or the interaction of ApoE with C1q or of ApoE with Aβ or complement component C5 or of complement component C5 or its cleavage products C5a and C5b or variants thereof or any complement constituent that arises between the C1q complex and C5 or cleavage products thereof or complement receptors on any cell or other genes outside of the complement cascades.

Just by way of example, small interference nucleotide molecules may be mentioned here as particularly preferred compounds for use in the present invention. However, any compound capable of fulfilling the functions defined above is also suitable.

Suitable small interference nucleotide molecules are e.g. agents developed by Alnylam Pharmaceuticals Inc. and described i.a. in US 2016/0244763, US 2015/0247143, WO 2016/201301, WO 2016/044419, EP 3034510 and US 2017/183659 to which reference is made for further details. The skilled person is aware of suitable siRNA agents and will select the suitable agent based on his professional knowledge and the specific application case.

By modulating the expression, function or stability of the interaction of ApoE with C1 q or with Aβ or of complement component C5 or its cleavage products C5a and C5b or variants thereof or of any complement constituent or their receptors arising between C1q and C5 in tissues, the unresolved inflammatory condition can be regulated and controlled, respectively, reduced or - under distinct disease conditions - enhanced.

In accordance with an embodiment of the present invention, the unresolved inflammatory condition is an aging-associated disease, in particular AD or other forms of dementia or neurodegenerative diseases including vascular dementia or atherosclerosis or ChP injury of patients with cognitive decline. An aging-associated disease is a disease that is most often seen with increasing frequency with increasing senescence. Essentially, aging-associated diseases are complications arising from senescence. Age-associated diseases are to be distinguished from the aging process itself because all adult beings age, save for a few rare exceptions, but not all adult beings experience all age-associated diseases.

Examples of aging-associated diseases are atherosclerosis and cardiovascular disease, cancer, many forms of arthritis, cataracts, AMD, osteoporosis, type 2 diabetes, hypertension, ChP injury of patients with/without cognitive decline, and AD and many other forms of dementia and cognitive decline. The compounds for use in the present invention may in particular be used for the treatment and diagnosis of atherosclerosis or AD or other diseases associated with cognitive decline or unresolved inflammation.

In the present invention it was recognized for the first time that C1q and Aβ form complexes with ApoE in vivo in AD plaques (C1q-ApoE complexes and Aβ-ApoE complexes, respectively) as evidenced by the proximity ligation assay (PLA) method. Both C1q-ApoE and Aβ-ApoE complexes constitute hitherto unknown pathological characteristics of AD plaques. Consequently, any diagnostic procedures to image these complexes or modulate the interaction of the two binding partners are also part of the present invention.

The C1q-ApoE complex has a modulating and activity-regulating effect on the complement cascade which could be evidenced by the successful treatment using C5 siRNA molecules and by experimental data of various transgenic ApoE knockin mice. The C1q-ApoE complex was identified in diseased ChPs, AD plaques, other brain areas, and atherosclerosis and many other unresolvable inflammatory diseases whereas the Aβ-ApoE complex was found in AD brains. Most if not all chronic inflammatory diseases are associated with activation of one or more complement pathways (Hajishengallis et al., 2017).

Each of the binding partners of the C1q-ApoE complex or the Aβ-ApoE complex may be subject to separate regulatory events, depending on multiple variables including the state of the immune system, age, the type of the injurious agent, and territorialized tissue contexts. These conditions may ultimately determine the concentration and impact of the C1q-ApoE complex or the Aβ-ApoE complex in specific disease conditions. It is believed that the C1q-ApoE complex forms an active disease-relevant regulatory module as indirectly evidenced by the frequent occurrence of autoimmune diseases or immune deficiencies in patients afflicted with genetic absence or loss of function mutations in C1q, C2, C4, and other components of the CCC (Hovland et al., 2015; Zhang et al., 2013).

Another embodiment of the present invention relates to a method for identifying a compound suitable for the prevention or treatment of a disease characterized by an unresolved inflammation condition comprising the step of contacting at least one of C1q or the interaction of ApoE with C1q or of ApoE with Aβ or the complement component C5 or its cleavage products C5a and C5b or variants thereof and/or a cell expressing C1q, complement component C5 or its cleavage products C5a and C5b or variants thereof or of any other complement constituent that arises between the C1q complex and C5 or cleavage products thereof or complement constituent receptor expressed on any cell, with at least one compound that potentially modulates the interaction of ApoE with C1q or Aβ, preferably the interaction of ApoE with C1q in a cell or outside of the cell or which potentially modulates the expression of complement component C5 or its components C5a and C5b or variants thereof in a cell or extracellularly in tissues, and thereafter identifying a modulation of the interaction of ApoE with C1 q or Aβ in a cell or tissues or of the expression of complement component C5 or its components C5a and C5b or variants thereof in a cell or tissue in the presence of said at least one compound.

Another embodiment of the present invention relates to an isolated antigen-binding construct capable of specifically binding to an interaction of ApoE with C1q or ApoE with Aβ or capable of modulating the expression of complement component C5 or its components C5a and C5b or variants thereof wherein said antigen-binding construct or variants thereof inhibits or enhances the expression of complement component C5 or its components C5a and C5b or variants thereof or of other complement pathway-related molecules which are modified as a result of the modulation of the C1q-ApoE or Aβ-ApoE complex.

Such antigen binding construct enables the control of the level of C1q-ApoE complex or of the Aβ-ApoE complex and thereby the control and regulation of the diseases indicated above for which the C1q-ApoE complex or the Aβ-ApoE are important indicators.

A still further embodiment of the present invention relates to a pharmaceutical composition comprising a compound for use in the treatment or prevention of a disease or condition in a mammalian subject wherein the compound is a modulator of the expression, function or stability of C1q or of the interaction of ApoE with C1q or with Aβ or of complement component C5 or its cleavage products C5a and C5b or variants thereof or of any other complement constituent or complement constituent receptor expressed on tissue or immune cells. The pharmaceutical composition does also contain a pharmaceutically acceptable carrier, stabilizer or excipient. Suitable carriers, stabilizers and excipients for pharmaceutical compositions have been described elsewhere in the literature and the skilled person will select the suitable components of the composition based on his professional expertise and the individual application case.

An excipient is a substance formulated alongside the active ingredient of a medication, included for the purpose of long-term stabilization, bulking up solid formulations that contain potent active ingredients in small amounts (thus often referred to as "bulking agents", "fillers", or "diluents"), or to confer a therapeutic enhancement on the active ingredient in the final dosage form, such as facilitating drug absorption, reducing viscosity, or enhancing solubility. Excipients can also be useful in the manufacturing process, to aid in the handling of the active substance concerned such as by facilitating powder flowability or non-stick properties, in addition to aiding in vitro stability such as prevention of denaturation or aggregation over the expected shelf life.

Common excipients are antiadherents, binders, coatings, colors, disintegrants, flavors, glidants, lubricants, preservatives, sweeteners, and sorbents which may be mentioned as examples.

The pharmaceutical compositions in accordance with the present invention can be used in a method for treating or preventing diseases or conditions involving unresolved inflammation in a patient wherein an effective amount of said pharmaceutical composition is administered, preferably in methods where the disease or condition is an aging-associated disease, in particular ChP injuries, AD or atherosclerosis.

C1q-ApoE complex formation or the Aβ-ApoE complex may be evaluated by the so called proximity ligation assay (PLA). Using PLA it was observed that the C1q-ApoE complex forms in human ChPs *in vivo* and that its density in ChP lipid-rich areas is higher when compared to lipid-free areas. Furthermore, the Aβ-ApoE complex was identified as a pathological hallmark of AD plaques. In the course of the present invention it has been found that the interaction product of ApoE with C1q (hereinafter referred to as the C1q-ApoE complex) in the ChP correlates well with lipid deposits in the choroid plexus which in turn correlates with cognitive decline in age-associated diseases, in particular with AD. Furthermore, the complex correlates with atherosclerosis in patients afflicted with carotid artery atherosclerosis.

C1q-ApoE complexes formed in vivo also may serve as a control monitor for atherosclerosis inflammation and in general for all unresolvable inflammatory diseases. Imaging of the complex or the Aβ-ApoE complex and/or lipid and/or lipid-derivatives and/or lipid-associated inflammation-derivatives (cells, proteins, peptides, miRNAs, and others) in the choroid plexus or in the brain of patients afflicted with cognitive decline or dementia using a variety of methods including antibodies is a new diagnostic tool to study the extent and the prognosis of the diseases.

Another embodiment of the present invention thus relates to the use of a complex being the reaction product of ApoE with C1q or with Aβ for the diagnosis of disease conditions associated with an unresolvable inflammation, in particular for the diagnosis of AD and other forms of dementia and atherosclerosis. The present invention provides for the first time the possibility to diagnose AD or atherosclerosis through the monitoring of the complexes formed by ApoE with C1q in a living mammalian subject. In particular, it has been found in the course of the present invention that lipid deposits in the choroid plexus correlates with the presence of the C1q-ApoE complex which in turn correlates with cognitive decline. Therefore, any diagnostic method that can be performed in living organisms and patients related to lipid and/or lipid-derivatives or calcium deposits, the presence of the C1q-ApoE complex or inflammatory infiltrates in the choroid plexus is of significant value to diagnose future or present cognitive decline or the clinical outcome of atherosclerosis. Moreover, the choroid plexus is a metabolically particularly active tissue which expresses a number of molecules in a concentrated form. These characteristics of the choroid plexus are suggested to form the basis for imaging technologies that target these characteristics or molecules. Before the present invention, in particular AD and other forms of dementia could only be diagnosed with high certainty and quantitative parameters post-mortem; the current diagnostic methods are based on qualitative factors but not on the quantitative measurement of a marker for the development and degree of the diseases. The present invention provides for the first time a quantitative correlation between the level of an identified marker (the C1q-ApoE complex or lipid or inflammatory markers in the choroid plexus) and the degree of development of the disease including cognitive decline. By measuring the level of the complex-associated pathologies including inflammatory cells, lipid, or calcium deposits in the choroid plexus or other parts of the brain including meninges or in peripheral arteries in atherosclerosis, the predisposition of a given mammalian subject to age-associated diseases like AD and atherosclerosis is possible. This opens the possibility for anticipatory and preventive therapies of the said diseases by identifying indirectly the complex levels and based on said determination, to take the necessary steps to regulate the concentration of the C1q-ApoE complex within the desired range or otherwise specifically treat the disease as outlined above targeting any complement constituent or complement constituent receptor on tissue and immune cells.

A still further embodiment of the present invention relates to a diagnostic method for the diagnosis of aging-associated diseases, in particular AD and atherosclerosis, wherein the level of lipid, inflammatory cells, and other structural or functional changes in the choroid plexus or other inflammatory tissue reactions including calcium deposits are monitored by an imaging method in vivo.

The choroid plexus can be imaged by a variety of methods with various markers or labels, such as Sestamibi, radiolabeled glucose, other radiolabeled compounds assessing various forms of metabolism, labeled immune cells, and various other imaging technologies. Sestamibi is a coordination complex consisting of the radioisotope technetium-99m bound to six (sesta=6) methoxyisobutylisonitrile (MIBI) ligands.

The imaging method may vary depending on the underlying disease but it may be a radiolabeled marker of metabolism (for example glucose) or an antibody directed against a glucose transporter. The choroid plexus is one if not the most metabolically active tissue in the mammalian body expressing hormone binding protein and transporters, ion channels, glucose transporters and many more (Lun et al., 2015; Schwartz and Baruch, 2014). Any compound that can image such highly expressed molecules in the choroid plexus is a candidate for disease diagnostics in the brain and in the choroid plexus in particular.

As mentioned above, C1q-ApoE or Aβ-ApoE complex formation can be evaluated using the PLA ex vivo, which is a technique known to the skilled person and which has been described in the literature so that no detailed description needs to be given here.

In the course of the present invention, it has also been found that the accumulation of lipid in the choroid plexus correlates with the degree of development of the diseases which may be monitored through the C1q-ApoE complex or other inflammatory markers in choroid plexus. This provides another possibility for a diagnostic method for the diagnosis of aging-associated diseases, in particular cognitive decline (including AD) and atherosclerosis, wherein the level lipid accumulation or any other change in the choroid plexus including a highly expressed molecule or an inflammatory infiltrate is monitored by an imaging method. Suitable imaging methods for the determination of lipid levels have been described in the literature and are known to the skilled person so that no detailed description needs to be given here.

Similar amounts of lipid accumulated in aged ApoE^{-/-} and high-fat diet-fed (HFD) ApoE4-knockin (ApoE4-KI) ChPs but no lipid accumulated in normal chow-fed (NC) ApoE4-KI or in NC or HFD ApoE3-KI ChPs (see Figures 1a-c).

Lipid deposits colocalized with leukocytes in ApoE-/- ChPs with the majority of macrophages/dendritic cells (DCs), which were increased in number by a factor of ∼15 (see Figures 1d and 1e). ChP leukocytes (Figure 1f), endothelial cells (Figure 1f), and epithelial cells (Figure 2a) accumulated intracellular lipid droplets, as did the ependymal cells lining the ventricle surfaces (Figure 2b). The adjacent brain parenchyma underneath the ependymal cells was infiltrated by lipid and leukocytes and exhibited signs of astrocyte activation. Extracellular lipid increased in ApoE^{-/-} versus WT ChPs by ∼18-fold and also localized at the luminal side of the epithelial cells (Figure 2a). High-resolution and transmission electron microscopy (TEM) revealed leukocytes/macrophages in the CSF attached to the microvilli at the abluminal side of the ChP; and some of the intraventricular macrophages accumulated lipid yielding a foam cell-like appearance (Figure 2c). These data suggest that macrophages on both sides of the blood-CSF barrier engulfed lipid. Extracellular ChP lipid appeared at the luminal side and the stromal space, which could be indicative for the possibility that immunoglobulins (Igs) bind to the lipid droplets. In ApoE^{-/-} ChPs, Igs colocalized with lipid inside the capillary lumen, the stromal space, and the lipid between the epithelial cells but no Ig binding occurred in lipid-free ChPs (see Figure 1f and Figure 2d). Bell et al. previously reported that ApoE-deficiency and transgenic expression of ApoE4 in NC ApoE4-KI mice were afflicted with BBB breakdown. Igs, which we used as a marker of BBB breakdown, accumulated in the perivascular space of the lipid-free brain parenchyma of ApoE-/- and NC or HFD ApoE4-KI mice. However, there was no statistically discernable aggravation of BBB dysfunction as a function of hyperlipidemia (Figure 2e).

To delineate differential effects of mouse ApoE vs human ApoE isoforms and the effects of hyperlipidemia on ChP gene expression, laser capture microdissection-based MIAME-compliant microarrays (http://www.ncbi.nih.gov/geo, the NCBI omnibus (GEO); accession: GSE85781) from ChPs of various mouse genotypes that had been maintained on NC or HFD were examined. 241 differentially expressed ChP genes in 6 transcriptomes were identified in gene ontology (GO) terms immune system process, transcription factor binding, cell junction, and ATP binding (Figure 2f). In ApoE-/- ChPs, the majority (81%) of differentially expressed genes were down-regulated when compared to WT (wild-type) ChPs (Figure 1g); however, 58% (7/12) of upregulated genes were interferon (IFN)-related genes with none downregulated (Figure 1g). Surprisingly, NC ApoE4 replacement ChPs further induced (44%, 22/50) IFN- related genes (Figure 1g). Multiple two-group comparisons revealed a pronounced ApoE4- specific ChP IFN signature (Figure 1g). The biological activities of the IFN-related genes range from regulation of autoimmunity by macrophages and DCs to BBB integrity including IFN-induced protein with tetratricopeptide repeats 3 and 1 (ifit3, ifit1), ubiquitin-specific peptidase 18 (usp18), guanylate-binding protein 3 (gbp3), interferon-induced protein 44 (ifi44), receptor transporter protein 4 (rtp4), IFN-regulatory factor 7 (irf7), and interferon, alpha-inducible protein 27 like 2A (ifi27l2a) (see Figure 1h). These data provided evidence for a detrimental and isoform-specific impact of ApoE4 in ChP homeostasis as ChP IFN has been associated with cognitive decline. Moreover, several genes that were down-regulated in ApoE-/- ChPs were rescued in their ApoE-KI counterparts, indicating phenotypic ChP changes specific for the ApoE-deficiency (Figure 2g). In addition, complement genes were up-regulated in ApoE-/- ChPs (Figures 3 and 4).

It is assumed that lipid deposits in ApoE^{-/-} ChPs bind Igs and thereby activate complement. Igs, C3, C3a, and C5 were evident together with lipid in ChPs of ApoE^{-/-} but not in WT mice (Figures 3a and b). Moreover, the CCC-initiating C1q complex and C4 colocalized with ChP lipid deposits (Figure 4a). Most complement constituents are produced by the liver and released into the circulation as inactive components or can be produced locally in peripheral tissues. C5 transcripts were below the threshold level in ChP transcriptomes, indicating that ChP C5 was serum/liver-derived (not shown). To examine whether ChP lipid-triggered CCC activation participates in leukocyte infiltration, we chose to specifically target liver-derived C5 using an si-RNA that selectively binds to the liver asialoglycoprotein receptor (Figure 3c). Liver C5-si-RNA knockdown led to a large decrease of circulating C5 levels without affecting blood lipoprotein concentrations or body weight (Figure 3c). Liver-targeted C5 silencing resulted in substantial decrease of C5 deposits in the ChP (Figure 4b) and attenuated CD45⁺ leukocyte-, CD68⁺ macrophage-/DC-, and CD3⁺ T-cell infiltration in ApoE^{-/-} ChPs (Figures 3d to f). In contrast, IgG, C4, and C3 deposition were much less affected (figures 4b and 4c). These data demonstrate that lipid-triggered complement cascade activation promoted ChP leukocyte infiltration. However, C3 and C4 were present at much lower levels in HFD ApoE4-KI ChPs *vs* ApoE^{-/-} ChPs (Figure 3g) despite similar amounts of ChP lipid (Figure 1c) and respective serum C3 and C5 levels (Figure 4d). ApoE colocalized with Igs and C1q Figure 4e, Figure 3h). Using an unbiased gene expression approach, i.e. expression microarrays, all complement-related genes signatures in ChPs were examined. Six transcripts encoding CCC-specific constituents (*c1qa*, *c1qb*, *c1qc*, *c2, c3ar1*, *c1ra*) were identified which were selectively upregulated in ChPs of ApoE^{-/-} as compared to WT mice (Figure 3i, Figure 4f). While factor H (alternative complement pathway inhibitor) mRNA was detectable without differences between groups (Figure 4g), factor B and MASP1 transcripts were below threshold levels. However, prominent factor H protein accumulation was observed on lipid deposits of both ApoE^{-/-} and HFD ApoE4 ChPs (Figure 4h), indicating the presence of a C3b-initiated amplification loop that was inhibited by factor H in both groups of mice. Interestingly, *c1qa* and *c1qc* transcripts were rescued in ApoE-KI *vs* ApoE^{-/-} ChPs (Figure 3i) and various complement regulators were expressed in ApoE^{-/-} and ApoE-KI ChPs (Figure 4f). Taken together, these data revealed pronounced CCC activation in ApoE^{-/-} but not in HFD ApoE3-KI and less in HFD ApoE4-KI mice. In addition, we found that ApoE mRNA ranges in the top 50 of ∼16 000 genes expressed in WT ChPs indicating that ApoE is expressed at extraordinary high levels in normal ChPs.

ChP lipid deposits correlate with cognitive decline in AD. Though ChP lipid deposits have not been reported in AD, it was searched for pathologies in human AD ChPs that may resemble the pathology of ApoE^{-/-} and HFD ApoE4-KI ChPs. 30 age- and gender-matched brains afflicted with various stages of AD-associated pathologies, i.e., Braak & Braak stages for neurofibrillary tangles (NFTs), Thai phase for Aβ plaque score, and the Consortium to Establish a Registry for Alzheimer's Disease (CERAD) for neuritic plaque (both NFTs and Aβ plaques) burden were investigated. 13/30 patients had no signs of dementia (Braak & Braak 0-III, Thai phase 0-5, CERAD stage 0), whereas 17/30 patients exhibited dementia upon clinical neurological examination and showed marked AD pathologies (Braak & Braak IV-VI, Thai phase 1-5, CERAD stage B-C). Surprisingly, 29 of the 30 brains showed various degrees of ChP lipid deposits that were strikingly similar to those found in ApoE^{-/-} and HFD ApoE4-KI ChPs (Figure 5a). Notably, demented AD cases revealed higher rates of lipid in ChPs versus non-dementia cases (Figure 5a). Moreover, the burden of ChP lipid deposits correlated with all AD neuropathologies (Figures 5b to 5d) and ChP lipid content especially correlated with ApoE4 allele carriers (Figure 5e). Unexpectedly, ApoE3/ApoE3 demented AD cases also had a significantly higher rate of ChP lipid positive areas when compared to ApoE3/ApoE3 non-dementia cases (Figure 5f). ChP lipid colocalized with C1q, ApoE, and complement C3 and C5 (Figure 5g, Figures 6a and 6b). ChP lipid deposits were associated with intraluminal macrophage infiltration, very similar to mouse ApoE^{-/-} ChPs (Figures 6c, 6d). Factor H protein deposition was observed in both lipid positive and lipid negative ChPs in dementia cases.

C1q-ApoE complexes in ChP and AD. A major question that arose from these data was whether the C1q-ApoE complex that was observed *in vitro* is also formed *in vivo.* C1q-ApoE complex formation was evaluated using the proximity ligation assay (PLA) with a resolution power of 10-30nm, comparable to resonance energy transfer-type technologies and super-resolution stimulated emission depletion (STED) microscopy was applied in parallel (Figure 5h). STED is a deterministic functional technique that exploits the nonlinear response of fluorophores commonly used to label biological samples in order to achieve an improvement in resolution, that is to say STED allows for images to be taken at resolutions below the diffraction limit. By PLA, we observed that the C1q-ApoE complex forms in human ChPs of demented AD cases *in vivo* (Figure 5i) and that its density in ChP lipid-rich areas was higher when compared to lipid-free areas (Figure 5i). C1q, Aβ, phosphorylated Tau (pTau), as well as C3 co-localized with ApoE (Figures 6f, g and h). C1q-ApoE complexes were also observed in human neuritic plaques (Figure 5j). Moreover, PLA revealed Aβ-ApoE complexes but not ApoE-pTau complexes in AD plaques of demented cases, extending and corroborating findings that ApoE does not bind to pTau *in vitro.* These data demonstrate the formation of the C1q-ApoE complex in diseased human brains of AD cases with dementia. C1q-ApoE complexes were also present in mouse AD pathologies (Figure 6h).

We find that C1q-ApoE complexes are involved in AD pathologies by using mouse models of AD or human brains. Using APPPS1-21 mice carrying a double mutation in the amyloid β and presenilin genes, these mice develop a rapid and severe form of AD pathology during a defined time window early after birth. C1q-ApoE complexes, but not Aβ-ApoE complexes were observed in 8 weeks old WT brain cortexes (Figure 7a). These data showed that C1q-ApoE complexes are not limited to inflammation, but may play a role in normal brain homeostasis, i.e. synaptic pruning. In 16-weeks old APPPS1-21 mouse brain cortexes, C1q-ApoE complexes were observed to be enriched in methoxy-X04+ Aβ plaques (Figure 7b). High resolution 3D confocal microscopy of the complexes revealed that they locate both inside and outside of Aβ plaques. Similar to our report in human AD brain, Aβ-ApoE complexes were also observed in APPPS1-21 mouse brain Aβ plaques as shown by 3D high resolution microscopy revealinging that the majority of Aβ-ApoE complexes locate inside methoxy-X04+ Aβ plaques (Figure 7c). Our data suggest that C1q-ApoE and Aβ-ApoE complexes emerge as hallmark for Aβ pathologies, and therefore may be involved in mediating AD pathology. Liver-specific C5 si-RNA treatment reduces serum C5 protein levels in the AD mouse model (Figure 8a). As C5 has a molecular weight of > 150 kD, we assume that it crosses the blood brain barrier in compromised mice, co-stain of blood vessels with CD31, Ig, and methoxy-X04 for Aβ plaque further support the BBB breakdown in AD mice (Figure 8b). C5 siRNA treatment significantly reduces the number and density of Aβ-associated microglia cells, Aβ plaque-associated LAMP1 and leads to a moderate reduction of intermediate sized plaque volumes (Figure 8c-f). These data revealed that liver-derived circulating C5 contributes to Aβ plaque inflammation.

C1q-ApoE complexes control atherosclerosis inflammation. The data obtained raised the possibility that other unresolvable human diseases showed similar pathological hallmarks that were identified in ApoE^{-/-} ChPs in mice and human AD brains. To assess this possibility, atherosclerosis was examined in ApoE^{-/-} mice as 9 complement pathway-related transcripts were found to be >2-fold upregulated in ApoE^{-/-} aortas during development of aortic arch atherosclerosis were found. Strong up-regulation of CCC complement genes in the aorta during early atherogenesis was observed (Figure 9a, Figures 10a and b). The impact of CCC activation on early atherosclerosis in ApoE^{-/-} mice was supported by a 65% decrease in both thoracic and abdominal aorta atherosclerosis by C5 siRNA (Figures 9b to 9e), without affecting blood lipid levels, body weight, or blood leukocyte counts (Figures 10c to 10g). CCC activation in human carotid atherosclerosis was examined. Five healthy control arteries on autopsy (type 0-I; American Heart Association classification), six early (type II-III) and nine advanced atherosclerotic plaques (type V-VII) from carotid endarterectomy specimens were stained for CD68⁺ macrophages/DCs, C1q, ApoE, and C5. CD68⁺ macrophages, and C1q, ApoE, and C5 protein deposits increased in early and advanced plaques when compared to control arteries (Figure 9g). C1q/ApoE co-localization was confirmed by STED microscopy (Figure 10h). Finally, the C1q-ApoE complex was identified as a pathological hallmark of atherosclerotic plaques (Figure 9h) and malondialdehyde-epitopes (MDA2) were observed on the surface of lipid deposits within plaques (Figure 9i).

In the course of the present invention, a CCC activity-stabilizing C1q-ApoE complex in diseased ChPs, AD plaques, and atherosclerosis was identified. ApoE controls tissue inflammation by binding to and blocking of C1q resulting in taming of the CCC. As ApoE qualifies as a physiological and direct regulator of complement, inappropriate control of the CCC by ApoE would be predicted to cause CCC malfunction, injurious tissue inflammation, and disease. The chronicity of unresolvable diseases indicates unrelenting overactivation of the CCC as an important pathogenic mechanism. That ApoE is indispensable for the regulation of CCC-dependent inflammation is indicated by the striking ChP and atherosclerosis pathologies, the presence of C1q-ApoE complexes in AD brains and atherosclerotic arteries, and the attenuation of inflammation by a C5-directed therapeutic in murine models of ChP inflammation, AD and atherosclerosis (Figures 8 and 9j).

Inflammatory mediators such as Aβ fibrils, oxLDL, C-reactive protein, and a large number of agents carrying danger-associated molecular patterns trigger the innate immune system via activation of C1q. The resulting CCC activity is controlled by a series of physiologically important inhibitors such as C1inh and C4bp. Most if not all chronic inflammatory diseases are associated with activation of one or more complement pathways and ApoE is induced in response to multiple acute and chronic types of tissue injury. In the course of the present invention, both C1q and ApoE independently emerged as mediators of innate immunity. Their ancestors can be traced to unicellular organisms where they participate in defense mechanisms. Both C1q and ApoE acquired domains that appear to have emerged from evolutionary pressures as mirrored by separate but also shared binding partners. For example, both C1q and ApoE bind to lipid- and microbial surfaces, Aβ fibrils, and heparan sulfate proteoglycans. It is assumed that C1q and ApoE are induced in multiple inflammatory microenvironments and that the C1q-ApoE complex may be a ubiquitous feature of inflammation.

Each of the two binding partners of the C1q-ApoE complex may be subject to separate regulatory events, however, depending on multiple variables including the state of the immune system, age, the type of the injurious agent, and territorialized tissue contexts. These conditions may ultimately determine the concentration and impact of the C1q-ApoE complex in specific disease conditions. Moreover, AD and atherosclerosis share risk factors such as hyperlipidemia, hypertension, obesity, and diabetes mellitus while the second most common form of dementia, i.e. vascular dementia, has been closely related to LOAD. Indeed, the incidence of AD is greatly enhanced in patients with atherosclerosis consistent with common mechanisms of disease progression.

The C1q-ApoE complex does not directly address the surprising effect of the ApoE4 isoform *vs* the ApoE2 and ApoE3 isoforms in LOAD or the differential effects of distinct ApoE isoforms in human atherosclerosis. Lipid deposits were identified in the ChPs of ApoE^{-/-} and HFD ApoE4 replacement mice but no lipid deposits or apparent CCC activities were detectable in either ND ApoE4 or ND ApoE3 replacement ChPs, yet their IFN gene expression signatures are dramatically different. These data show a unique hitherto overlooked ApoE isoform-specific ChP pathology that is likely to impact brain homeostasis and AD pathogenesis and that the ChP gene signature or single genes thereof may turn out to be a therapeutic target in AD and vascular dementia.

The assumption that the C1q-ApoE complex forms an active disease-relevant regulatory module is consistent with the frequent occurrence of autoimmune diseases or immune deficiencies in patients afflicted with genetic absence or loss of function mutations in C1q, C2, C4, and other components of the CCC and the identification of both complement and ApoE as major players in LOAD and atherosclerosis.

97% (29/30) of our aged human cohort show various degrees of ChP lipid deposition, which was correleated with cognitive decline, the ApoE4-isoform, and brain AD-related pathologies. These data is consistent with the possibility that ChP injury occurs earlier than brain Aβ-piaque formation or tau pathologies, indicating that ChP lipid-assocaited pathologies may represent pre-events of cognitive decline in humans. Lipid deposits were identified in the ChPs of ApoE^{-/-} and high fat diet-fed (HFD) ApoE4 mice but no lipid deposits were detectable in either normal chow (NC) ApoE4 or HFD ApoE3 ChPs, suggesting ApoE4-isoform together with hyperlipidemia caused ChP lipid deposition in mice. Indeed, LOAD and atherosclerosis share risk factors such as hyperlipidemia, hypertension, obesity, and diabetes mellitus, and the incidence of LOAD is greatly enhanced in patients with atherosclerosis consistent with common mechanisms of disease progression. Moreover, our data raise the possibility to diagnose and prevent the cognitive decline in patients before the irreversible Aβ- and tau-pathologies occur in human brains, and suggest novel therapeutic strategies for LOAD, as well as atherosclerosis.

### References:

Bell, R.D., Winkler, E.A., Singh, I., Sagare, A.P., Deane, R., Wu, Z., Holtzman, D.M., Betsholtz, C., Armulik, A., Sallstrom, J., et al. (2012). Apolipoprotein E controls cerebrovascular integrity via cyclophilin A. Nature 485, 512-516.
Brennan, F.H., Lee, J.D., Ruitenberg, M.J., and Woodruff, T.M. (2016). Therapeutic targeting of complement to modify disease course and improve outcomes in neurological conditions. Sem Immunol.
Hajishengallis, G., Reis, E.S., Mastellos, D.C., Ricklin, D., and Lambris, J.D. (2017). Novel mechanisms and functions of complement. Nat Immunol 18, 1288-1298.
Hess, C., and Kemper, C. (2016). Complement-mediated regulation of metabolism and basic cellular processes. Immunity 45, 240-254.
Hofman, A., Ott, A., Breteler, M.M., Bots, M.L., Slooter, A.J., van Harskamp, F., van Duijn, C.N., Van Broeckhoven, C., and Grobbee, D.E. (1997). Atherosclerosis, apolipoprotein E, and prevalence of dementia and Alzheimer's disease in the Rotterdam Study. Lancet 349, 151-154.
Holtzman, D.M., Herz, J., and Bu, G. (2012). Apolipoprotein E and apolipoprotein E receptors: normal biology and roles in Alzheimer disease. Cold Spring Harbor perspectives in medicine 2, a006312.
Hovland, A., Jonasson, L., Garred, P., Yndestad, A., Aukrust, P., Lappegård, K.T., Espevik, T., and Mollnes, T.E. (2015). The complement system and toll-like receptors as integrated players in the pathophysiology of atherosclerosis. Atherosclerosis 241, 480-494.
Kanekiyo, T., Xu, H., and Bu, G. (2014). ApoE and Aβ in Alzheimer's disease: accidental encounters or partners? Neuron 81, 740-754.
Kolev, M., Friec, G.L., and Kemper, C. (2014). Complement - tapping into new sites and effector systems. Nat Rev Immunol 14, 811-820.
Kouser, L., Madhukaran, S.P., Shastri, A., Saraon, A., Ferluga, J., Al-Mozaini, M., and Kishore, U. (2015). Emerging and Novel Functions of Complement Protein C1q. Frontiers in Immunology 6*.*
Linton, M.F., Atkinson, J.B., and Fazio, S. (1995). Prevention of atherosclerosis in apolipoprotein E-deficient mice by bone marrow transplantation. Science 267, 1034-1037.
Lun, M.P., Monuki, E.S., and Lehtinen, M.K. (2015). Development and functions of the choroid plexus-cerebrospinal fluid system. Nat Rev Neurosci 16, 445-457.
Mahley, R.W. (1988). Apolipoprotein E: cholesterol transport protein with expanding role in cell biology. Science 240, 622-630.
Mahley, R.W., and Huang, Y. (2012). Apolipoprotein E sets the stage: response to injury triggers neuropathology. Neuron 76, 871-885.
Mahley, R.W., Weisgraber, K.H., and Huang, Y. (2009). Apolipoprotein E: structure determines function, from atherosclerosis to Alzheimer's disease to AIDS. Journal of lipid research 50 Suppl, S183-188.
Mohanta, S.K., Yin, C., Peng, L., Srikakulapu, P., Bontha, V., Hu, D., Weih, F., Weber, C., Gerdes, N., and Habenicht, A.J. (2014). Artery Tertiary Lymphoid Organs Contribute to Innate and Adaptive Immune Responses in Advanced Mouse Atherosclerosis. Circulation research 114, 1772-1787.
Plump, A.S., Smith, J.D., Hayek, T., Aalto-Setala, K., Walsh, A., Verstuyft, J.G., Rubin, E.M., and Breslow, J.L. (1992). Severe hypercholesterolemia and atherosclerosis in apolipoprotein E-deficient mice created by homologous recombination in ES cells. Cell 71, 343-353.
Schwartz, M., and Baruch, K. (2014). The resolution of neuroinflammation in neurodegeneration: leukocyte recruitment via the choroid plexus. EMBO J 33, 7-22.
Stevens, B., Allen, N.J., Vazquez, L.E., Howell, G.R., Christopherson, K.S., Nouri, N., Micheva, K.D., Mehalow, A.K., Huberman, A.D., Stafford, B., et al. (2007). The Classical Complement Cascade Mediates CNS Synapse Elimination. Cell 131, 1164-1178.
Zhang, B., Gaiteri, C., Bodea, L.G., Wang, Z., McElwee, J., Podtelezhnikov, A.A., Zhang, C., Xie, T., Tran, L., Dobrin, R., et al. (2013). Integrated systems approach identifies genetic nodes and networks in late-onset Alzheimer's disease. Cell 153, 707-720.
Zlokovic, B.V. (2013). Cerebrovascular effects of apolipoprotein E: implications for Alzheimer disease. JAMA neurology 70, 440-444.

## Claims

1. A compound for use in the treatment or prevention of a disease or condition in a mammalian subject wherein the compound is a modulator of the expression, function or stability of C1q or of the interaction of ApoE with C1q or of ApoE with Aβ or of complement component C5 or its cleavage products C5a and C5b or variants thereof or of any complement constituent that arises between the C1q complex and C5 or cleavage products thereof or complement receptors on any cell and wherein said disease is selected from unresolved inflammatory conditions.

2. The compound for use in accordance with claim 1 wherein the compound is an inhibitor or antagonist or agonist of said expression, function or stability of C1q or of the interaction of ApoE with C1q or of ApoE with Aβ or of complement component C5 or its cleavage products C5a and C5b or variants thereof or of any complement constituent that arises between the C1q complex and C5 or cleavage products thereof or complement receptors expressed on any cell.

3. The compound for use in accordance with any of claims 1 or 2 wherein said compound is an antigen-binding construct e.g. an antibody or an antibody-like molecule or other antigen-binding derivative or an antigen-binding fragment thereof that binds said C1q or said interaction of ApoE with C1q or of ApoE with Aβ or complement component C5 or its cleavage products C5a and C5b or variants thereof of any complement constituent that arises between the C1q complex and C5 or cleavage products thereof or complement receptors on any cell.

4. The compound for use in accordance with any of claims 1 to 3 wherein the compound is a nucleic acid, in particular an anti-sense nucleotide molecule or a siRNA or shRNA molecule that binds to a nucleic acid that encodes or regulates the expression of (i) C1q or the interaction of ApoE with C1q or of ApoE with Aβ or complement component C5 or its cleavage products C5a and C5b or variants thereof or of any complement constituent that arises between the C1q complex and C5 or cleavage products thereof or complement receptors on any cell; (ii) a gene that controls the expression, function and/or stability of C1q or the interaction of ApoE with C1q or complement component C5 or its cleavage products C5a and C5b or variants thereof of any complement constituent that arises between the C1q complex and C5 or cleavage products thereof or complement receptors on any cell.

5. The compound for use in accordance with any of claims 1 to 4 wherein the unresolved inflammatory condition is an aging-associated disease.

6. The compound for use in accordance with claim 5 wherein said aging associated disease is atherosclerosis or Alzheimer's disease (AD) or other diseases associated with cognitive decline or unresolved inflammation.

7. A method for identifying a compound suitable for the prevention or treatment of a disease **characterized by** an unresolved inflammation condition comprising the step of contacting at least one of C1q or the interaction of ApoE with C1q or of ApoE with Aβ or the complement component C5 or its cleavage products C5a and C5b or variants thereof and/or a cell expressing C1q, complement component C5 or its cleavage products C5a and C5b or variants thereof or any complement constituent that arises between the C1q complex and C5 or cleavage products thereof or complement receptors on any cell or a compound that potentially modulates the interaction of ApoE with C1q or Aβ in a cell or which potentially modulates the expression of complement component C5 or its components C5a and C5b or variants thereof in a cell, and thereafter identifying a modulation of the interaction of ApoE with C1q or Aβ in a cell or of the expression of complement component C5 or its components C5a and C5b or variants thereof or any complement constituent that arises between the C1q complex and C5 or cleavage products thereof or complement receptors on any cell in a cell in the presence of said at least one compound.

8. Use of a complex of any complement constituent that arises between the C1q complex and C5 or cleavage products thereof or complement receptors on any cell being the reaction product of an interaction of ApoE with C1q or of ApoE with Aβ for diagnosis of disease conditions associated with an unresolvable inflammation.

9. Use in accordance with claim 8 wherein the disease condition is AD or atherosclerosis or another condition associated with cognitive decline.

10. Diagnostic method for the diagnosis of aging-associated diseases, in particular AD and atherosclerosis or other forms of dementia or inflammatory brain disease, wherein the level of a complex resulting from the interaction of C1q with ApoE in the choroid plexus is monitored by an imaging method.

11. An isolated antigen binding construct capable of specifically binding to an interaction of ApoE and C1q or of ApoE with Aβ or capable of modulating the expression of complement component C5 or its components C5a and C5b or variants thereof of any complement constituent that arises between the C1q complex and C5 or its cleavage product or complement receptors on any cell wherein said antigen binding construct inhibits or enhances, inhibits or enhances, the expression of complement component C5 or its components C5a and C5b or variants thereof of any complement constituent that arises between the C1q complex and C5 or its cleavage product or complement receptors on any cell.

12. A pharmaceutical composition comprising a compound for use in accordance with any of claims 1 to 6 or an isolated antigen binding construct in accordance with claim 11 and a pharmaceutically acceptable carrier, stabilizer or excipient.

13. A method for treating or preventing diseases or conditions involving unresolved inflammation in a patient comprising administering to said patient an effective amount of a pharmaceutical composition according to claim 12.

14. The method according to claim 13 wherein the disease or condition is an aging-associated disease or condition is AD or atherosclerosis or other forms of dementia.

15. Diagnostic method for the diagnosis of aging-associated diseases, in particular AD and atherosclerosis or other forms of dementia, wherein the level lipid accumulation or inflammatory cell or other highly expressed genes including interferon-related genes in the choroid plexus is monitored by an imaging method or by determination of circulating molecules in the blood or/ cerebralspinal fluid (CSF) that are released by the diseased choroid plexus or the diseased brain or the diseased artery as a diagnostic procedure to diagnose atherosclerosis or choroid plexus inflammation or brain inflammation or any form of dementia.
